Europäisches Patentamt

European Patent Office

Office européen des brevets

(11) Publication number: **0 065 541**
**B1**

## (12) EUROPEAN PATENT SPECIFICATION

(45) Date of publication of patent specification: **18.09.85**

(21) Application number: **81903186.5**

(22) Date of filing: **12.11.81**

(86) International application number:
**PCT/US81/01509**

(87) International publication number:
**WO 82/01646 27.05.82 Gazette 82/14**

(51) Int. Cl.⁴: **A 61 B 17/30**, A 61 F 9/00

(54) INTRAOCULAR LENS FORCEPS.

(30) Priority: **13.11.80 US 206488**

(43) Date of publication of application:
**01.12.82 Bulletin 82/48**

(45) Publication of the grant of the patent:
**18.09.85 Bulletin 85/38**

(84) Designated Contracting States:
**AT CH DE FR GB LI LU NL SE**

(56) References cited:
**GB-A- 999 138**
**US-A-3 817 078**
**US-A-4 198 980**

(73) Proprietor: LeVEEN, Harry H.
321 Confederate Circle
Charleston, SC 29407 (US)
(73) Proprietor: RUBRICIUS, Jeanette L.
321 Confederate Circle
Charleston, SC 29407 (US)

(72) Inventor: LeVEEN, Harry H.
321 Confederate Circle
Charleston, SC 29407 (US)
Inventor: RUBRICIUS, Jeanette L.
321 Confederate Circle
Charleston, SC 29407 (US)

(74) Representative: McCall, John Douglas et al
W.P. THOMPSON & CO. Coopers Building
Church Street
Liverpool L1 3AB (GB)

Courier Press, Leamington Spa, England.

## Description

Background of the Invention

The condition of lenticular opacity in the eye, known as a cataract, is a leading cause of blindness and is especially common among the elderly. The primary means of alleviating a cataract is to remove the diseased natural lens, and more than 400,000 persons in the United States undergo surgery for removal of clouded lenses in eyes each year.

When no lens is present in the eye, which is known as an aphakic condition or aphakia and is usually the result of intracapsular or extracapsular lens extraction, the eye does not have the ability to focus rays of light. Therefore, the eye receives a blurred image and vision is severely impaired.

The most common solution for providing a focusing mechanism to obviate the aphakic condition is to interpose contact lenses or spectacles or a combination thereof between the eye and the light entering therein. However, both contact lenses and spectacles have drawbacks when used in the treatment of aphakia. Neither spectacles nor contact lenses can duplicate the natural optical system because they are positioned outside of the eye, which results in a shift of the optical center from the vivo state. Because the optical center has been shifted, the image received by the eye is changed in size.

Many aphakia patients who have had their cataracts removed are fitted with glasses or spectacles. These thick lenses present many more problems than they solve. Immediately upon receiving cataract spectacles a patient is confronted with several problems in that there is significant increase in the size and shape of familiar objects and straight lines are formed into curves.

Contact lenses are superior to thick cataract spectacles since the wearer enjoys good peripheral vision. The magnification problem does not bother contact lens wearers as much as it does cataract spectacle wearers, because the contact lenses only magnify in the range of 7 to 10 percent. Furthermore, hand to eye coordination of contact lens wearers is better than in spectacle wearers, as objects are seen in more normal spatial orientation and straight lines are not seen as curves. However, contact lenses are very small and fragile, and it is difficult to insert and remove them daily, particularly for elderly users or individuals with arthritis or coordination problems. In addition allergies and dry eye conditions also interfere with contact lens wearing.

The most promising method of sight restoration for cataract patients is the intraocular lens. An intraocular lens (hereinafter sometimes referred to as an IOL in various parts of the specification) is one which is placed inside the eye. When an IOL is implanted in substantially the same location formerly occupied by the natural lens, relatively normal vision may be restored to the patient. Generally lens implantation and cataract surgery takes around 45 minutes to an hour and with the lens implant the person usually has improved vision within a couple of days, and continued improvement over several more weeks until the eye is completely healed.

Intraocular lenses provide a significant improvement over the previously used artificial ocular aids in that once the implantation has been implemented patients regain a close approximation of their former visual function. The wearers of intraocular lens implants regain full side to side vision and problems of magnification and depth perception are practically non-existent. Since the intraocular lenses are permanently implanted within the eye, problems of daily cleaning, insertion and removal, and loss and replacement are eliminated. Furthermore, the wearer can enjoy sports such as swimming as the lenses remain in the eye and cosmetically there is no difference between persons who have intraocular lenses and those persons who have had no history of cataracts or eye surgery.

Examples of lenses which are placed in the anterior chamber and are secured to the iris by various methods are shown in United States Patent Nos. 3,673,611; 3,906,551; 3,922,728; 3,925,825; 3,971,073; 3,975,779; 3,979,780; 3,986,214; 3,996,627; 4,010,496; 4,056,855; 4,073,015; 4,077,071; 4,079,470; and 4,087,866.

Artificial lenses designed for positioning in the posterior chamber are described in United States Patent Nos. 3,711,870 and 4,014,049. The lenses disclosed in these patents comprise a central optical element surrounded by a resilient silicone flange shaped to receive and nest against the ciliary body. The lenses are held in place by suturing the resilient flange to the ciliary body. Another lens shown in U.S. Patent Nos. 3,925,835 and 4,014,089 is designed for implantation in either the anterior or posterior chamber of the eye, with the lens supporting (haptic) section of the IOL comprising a plurality of flexible spring like members designed to follow the margin of the dynamic pupil, while providing longitudinal fixation and centration of the lens. Patent Nos. 4,053,953 and 3,866,249 disclose a posterior lens held in place by an insertion necklace in the former and a holding ring in the latter. In U.S. Patent No. 4,041,552 the lens element is placed in the posterior chamber and supported by support on the anterior side of the iris, while a lower arm is sutured to the ciliary body and sclera at one side of the iris with another arm extending to the opposite side.

U.S. Patent No. 3,913,148 discloses a lens apparatus inserted in the posterior chamber, with a plurality of cantilevered clips, each of which is mounted to a central portion which extends outward from the face of the lens towards the periphery. The clips are used to secure the iris to the front face of the lens when the lens is positioned within the posterior chamber of the eye behind the iris.

Various lenses are discussed in an article by D.P. Choyce entitled "History of Intraocular Implants" which is printed in Annals of Opthal- mology, October 1973. The article also includes a list of references from which further information concerning prior art intraocular lenses can be obtained. The aforementioned prior art lenses represent typical lenses which can be used in the present lens holding apparatus.

In the performance of intraocular lens implantation surgery, it is of course necessary to place and orient the delicate plastic lens accurately at the implantation site. Typically, each intraocular lens is less than half the diameter of a contact lens and is correspondingly light and flimsier. Like contact lenses and spectacles, an intraocular lens may be subject to scratching of its surface which degrades its optical qualities, but unlike exterior lenses, the intraocular lens is not easily replaced when damaged.

An example of a lens holder for contact lenses in the prior art is found in United States Patent No. 3,063,083 and corresponding GB—A— 999138. In these references, the lens holder is used for immersing a contact lens in cleaning fluid. The jaws of the lens holder are lined with radial protuberances arranged in pairs spaced intermittently about the edges of the lens within the jaws. The circumference of the lens is engaged by the protuberances so that the periphery of the lens is separated from the jaws by clearance spaces. Thus, the minimal contact of the lens holder with the lens allows the greatest amount of lens surface area to come into contact with the cleaning fluid. However the pressure exerted on the lens, whilst acceptable in the context of the contact lenses being dealt with, is not acceptable in holding an intraocular lens, which offers less structural resistance to damage than a contact lens. Further if a device of these two specifications scaled down to a size appropriate to an intraocular lens, the sizes of jaws would be greater than is appropriate for a minimal surgical incision length on the eye.

United States Patent No. 3,817,087 discloses a wound clip removal device with jaws biased in a partly open positioned. The jaws may be opened further by placing pressure on two bowed handle portions which flex the jaws open.

Other patents of interest in the field of small clamping devices include United States Patent Nos. 1,521,689; 1,748,765; 2,222,744; 2,477,466; 2,595,683; 2,943,521; 3,063,083; 3,265,068; 3,650,008; 3,677,112; 3,817,078; 3,977,410; and 4,044,771. These devices share the deficiencies of numerous sharp and abrasive surfaces and a lack of adaptation for the particular nature of intraocular lenses.

Therefore, there is a clear need in the art for a means to hold an intraocular lens during surgery which will allow a lens to be oriented properly and accurately positioned either anterior or posterior to the iris, and which will hold the lens securely, yet without excess pressure which may damage the lens. The lenses generally have small loops which must be manoeuvered to engage them into proper position. The lens holder should firmly grasp the lens but not continuously squeeze it since pressure can induce a creep into plastic lens material which will distort the optical qualities of the lens. In addition, since there is little room in the anterior chamber to manipulate a lens, the neck of the inserting device must be kept small. Further, the holding means should maintain a constant grasp on the lens during manipulation by the physician, because small differences in tension and motion may result in dropping the lens onto an unsterile surface or a surface which scratches the lens or even losing grasp of the lens at a critical moment of implantation. Ordinary forceps have the disadvantage of requiring finger pressure to hold the lens, and movement of the jaws is magnified as compared to the movement of the compressing fingers. The lens holder should also be strong, light and of small dimensions so that it is easy to manipulate yet not so large as to require broad incision into the eye.

According to the present invention there is provided a combination of an intraocular lens having a predetermined diameter and a lens holder therefor said lens holder comprising first and second jaw members, a fulcrum, and a handle; each of said first and second jaw members being attached to said fulcrum and spaced apart from one another, said jaw members being arranged so as to selectively hold and release the lens, said handle comprising first and second extensions of said first and second jaw members beyond said fulcrum, said extensions being substantially identical to each other and spaced apart from one another for a predetermined distance from said fulcrum, said extensions being united at a distance from said fulcrum, each of said first and second jaw members defining an inner face, said inner faces being spaced apart a distance less than said diameter of said lens, each of said jaw members further defining an arcuate slot within said inner face retaining said lens without applying pressure thereto, said arcuate slots preferably being of a radius equal to the radius of said lens, said first and second extensions being coupled respectively to said first and second jaw members and sufficiently flexible so that finger compression of said extensions together separates said jaw members to release said lens held in said holder.

The pair of jaws are spaced apart a distance slightly less than the diameter of the lens to be held, and the surfaces of the jaws which face each other are slotted lengthwise so that when the jaws are in their normal position, a lens may be retained within the slotted faces without pressure. The neck of the holder is flattened in the same plane as the lens so that both may be inserted through a small surgical incision without difficulty.

The fulcrum normally comprises a small cross piece extending between the two jaws and behind the slotted jaw surfaces in which the lens is held. The jaws extend beyond the fulcrum to form a handle, and the extension of the jaws eventually unite at a point well beyond the fulcrum. The jaws and the handle preferably meet at the fulcrum at an angle to each other to allow placement of the lens without either prying the cornea or the iris during its insertion.

When the lens is placed at the implantation site, it may be released from the jaws by squeezing the extensions of the jaws behind the fulcrum. The jaws are forced apart through the action of the fulcrum allowing removal of the holder from the surgical site, leaving the lens behind.

The present invention may be made of one-piece disposable semi-rigid plastic or other smooth lightweight material, and has no jagged edges or surfaces to interfere with manipulation by the physician or to damage the eye or the lens itself. Being of one-piece construction, the present invention also offers the advantage of having no delicate small separable parts to lose or be dropped within or without the eye.

Typically, the present invention will be packaged in a sterile wrapping with a lens placed in the jaws. Therefore, the physician need not expose the holder or the lens to potentially destructive sterilization procedures immediately prior to implantation.

The above-mentioned purposes and operations of the invention are more readily apparent when read in conjunction with the following description of the drawings, and the detailed discussion of the preferred embodiment of the present invention.

Brief Description of the Drawings.

FIGURE 1 is a top plan view of the forceps of the invention showing the jaws in an open position;

FIGURE 2 is a top plan view of the apparatus of FIGURE 1 with the jaws shown in a closed position;

FIGURE 3 is a cross-sectional view of the apparatus of FIGURE 2 taken along line A—A'; and

FIGURE 4 is a side view of the apparatus of FIGURE 1.

Detailed Description of the Drawings

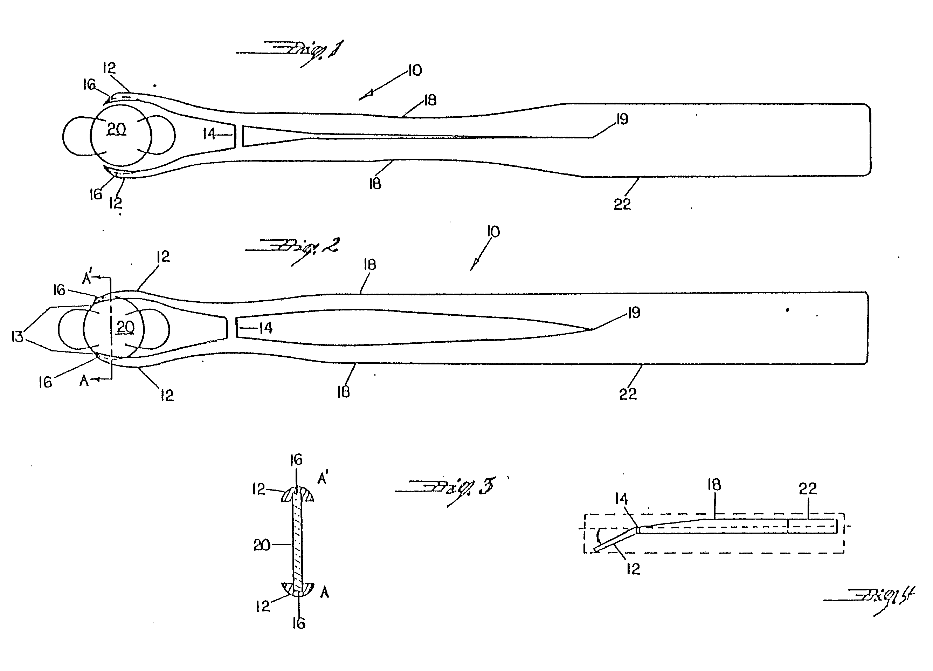

FIGURES 1 through 4 show the preferred embodiment and best mode of the invention. The lens holder, generally indicated at 10, comprises a pair of opposed jaws 12, a fulcrum 14 separating the jaws 12, and an extension 18 of each of the two jaws 12 extending away from the fulcrum 14. The two jaw extensions 18 meet to form handle 22 at an integral connection 19. The extensions 18 meet at a distance from fulcrum 14 which ranges from 3.175 to 4.445 cm (1.25 to 1.75 inches) and is preferably 3.4925 cm (1.375 inches). Thus, the two extensions 18 are separated from each other allowing finger pressure on the jaw extensions 18 to push the jaw extensions 18 toward each other into contact with one another.

The face of each opposing jaw 12 defines an arcuate slot 16, the radius of which corresponds to the radius of the intraocular lens intended to be held by the jaws 12. When the jaws 12 are in their normal closed position as is illustrated in FIGURE 2, a typical intraocular lens 20 may be held securely within the slots 16 of the jaws 12 without pressure on the lens 20. FIGURE 3 shows a cross-section of the jaws 12 and lens 20 taken at line A—A' of FIGURE 2, and shows that the portion of the jaws 12 which surround the slots 16 and the lens 20 prevents the lens 20 from moving to the right or to the left. Referring again to FIGURE 2, the mouth 13 of jaws 12 is normally open less than the diameter of lens 20, so that lens 20 may not fall out of the mouth.

FIGURE 1 shows the manner in which the lens 20 may be released from the jaws 12 when the lens is placed within the eye during a surgical operation. The jaw extensions 18 are depressed by finger pressure toward one another and may contact one another. Pressure on jaw extensions 18 rotates the jaws 12 around fulcrum 14, thus moving the slots 16 away from lens 20 and enlarging the mouth between the jaws 12 so that the diameter of lens 20 is less than the width of the mouth. Contact of the jaw extensions 18 against each other affords positive feedback to the surgeon that the lens has been released, and is not damaging to the lens 20 or the lens holder 10. Positive feedback of this sort is extremely valuable to a physician in surgical procedures such as intraocular lens placement where both the lens and the tissues involved are small, and the physician's ability to view the surgical site is limited.

Contact of the jaw extensions 18 also prevents jaws 12 from opening so wide as to damage sensitive eye tissue through which the lens holder 10 is moved during surgery.

FIGURE 4 shows the angle at which the jaws 12 are mounted with respect to the jaw extensions 18 and handle 22. The placement of jaws 12 at an acute angle to the line of jaw extensions 18 and handle 22 allows the physician to manoeuver more accurately within the confines of the human eye than would a straight lens holder. This angle preferably falls within the range of ten degrees to fifty degrees. The angled construction allows placement of the lens without prying either the cornea or the iris during its insertion.

The lens holder is typically formed by injection molding of a thermoplastic which is rigid or semi-rigid and sufficiently flexible to allow jaw and extension movement in the shape and size required. Such plastics include high density polyethylene, polypropylene, filled or unfilled polyacetyl, polystyrene, ABS polymers and nylon. In the preferred embodiment, the lens holder is made of fiberglass-filled polyacetyl. These materials give maximum strength with minimal volume, and allow the jaw extensions 18 to resist normal finger pressure and prevent accidental opening of the jaws. Thus, the lens holder thus gives the maximum finger control and requires sufficient force to open the jaws while being easy to manipulate with respect to rotation. Furthermore, the constructional distances of the lens holder are such that magnification of movement does not occur.

Typically, the lens holder 10 is sterilized immediately after manufacture and then packaged in an air-tight plastic envelope wrapper with a sterilized lens 20 placed between jaws 12. This enables the physician to avoid the step of placing or grasping a lens 20 within jaws 12 of the lens holder which could allow the lens to pop out of the holder in an undesirable location or cause damage to the lens.

While the preferred embodiment of the invention has been disclosed, it is understood that the invention is not limited to such an embodiment, since it may be otherwise embodied in the scope of the appended claims.

## Claims

1. A combination of an intraocular lens (20) having a predetermined diameter and a lens holder (10) therefor; said lens holder (10) comprising first and second jaw members (12), a fulcrum (14), and a handle (22); each of said first and second jaw members (12) being attached to said fulcrum (14) and spaced apart from one another, said jaw members (12) being arranged so as to selectively hold and release the lens, said handle comprising first and second extensions (18) of said first and second jaw members (12) beyond said fulcrum (14), said extensions (18) being substantially identical to each other and spaced apart from one another for a predetermined distance from said fulcrum (14), said extensions (18) being united at a distance from said fulcrum (14), each of said first and second jaw members (12) defining an inner face, said inner faces being spaced apart a distance less than said diameter of said lens, each of said jaw members further defining an arcuate slot (16) within said inner face retaining said lens (20) without applying pressure thereto, said first and second extensions (18) being coupled respectively to said first and second jaw members (12) and sufficiently flexible so that finger compression of said extensions (18) together separates said jaw members (12) to release said lens held in said holder.

2. A combination as claimed in claim 1, characterised in that said extensions (18) are united at a distance from said fulcrum (14) ranging from 3.175 to 4.445 cm (1.25 to 1.75 inches).

3. A combination as claimed in claim 2, characterised in that wherein said extensions (18) are united at a distance from said fulcrum (14) of 3.4925 cm (1.375 inches).

4. A combination as claimed in any one of claims 1 to 3 characterised by comprising integral end connector means (19) coupling said extensions (18) at respective ends opposite said jaw members (12), and wherein said extensions (18) are flexibly movable to contact one another between said fulcrum (14) and said integral end connector means (19), said contact area forming a stop to limit the maximum separation of said first and second jaw members (12).

5. A combination as claimed in any one of claims 1 to 4, characterised in that said first and second jaw members (12) intersect a line drawn through the axis of said extensions to define an acute angle at said fulcrum (14).

6. A combination as claimed in claim 5, characterised in that said acute angle ranges from ten degrees to fifty degrees.

7. A combination as claimed in any one of claims 1 to 6, characterised in that said lens holder (10) is an integrally formed apparatus.

8. A combination as claimed in any one of claims 1 to 7, wherein said jaw members (12) are curved toward each other so that the distance between the ends of the jaw members is less than the diameter of the lens (20).

9. A combination of an intraocular lens (20) of predetermined diameter and a lens holder (10) therefor, said lens holder comprising first and second jaw members (12), a fulcrum (14), and a handle (22); each of said first and second jaw members (12) being attached to said fulcrum (14) and spaced apart from one another, each of said first and second jaw members (12) defining an inner face, said inner faces of said first and second jaw members (12) being spaced apart a distance less than the diameter of intraocular lens (20), each of said inner faces further defining an arcuate channel (16) retaining said intraocular lens (20), without applying pressure thereto said handle (22) comprising first and second extensions (18) of said first and second jaw members, said extensions (18) having at least some portions spaced apart from one another, and extending away from said fulcrum (14) a distance ranging from 3.175 to 4.445 cm (1.25 to 1.75 inches), said extensions being connected together at a distance from said fulcrum (14) and defining an angle with said jaw members (12).

10. A combination as claimed in any one of claims 1 to 9, characterised in that said arcuate slots or channels (16) are of a radius equal to the radius of the intraocular lens (20).

## Patentansprüche

1. Eine Kombination einer Innenaugen-Linse (20), die einen vorgegebenen Durchmesser hat, und eines Linsenhalters (10) hierfür; genannter Linsenhalter (10) aufweisend erste und zweite Jochteile (12), ein Schwenklager (14) und einen Handgriff (22); jedes der ersten und zweiten Jochteile (12) angebracht an genanntem Schwenklager (14), wobei die Jochteile (12) in gegenseitigem Abstand so angeordnet sind, daß sie die Linse selektiv halten und freigegeben, wobei der Handgriff erste und zweite Verlängerungen (18) der ersten und zweiten Jochteile (12) jenseits des Schwenklagers (14) aufweist, wobei die Verlängerungen (18) im wesentlichen zueinander identische Ausbildung haben, über einen vorgebenen Abstand von dem Schwenklager (14) voneinander beabstandet und in einem Abstand vom Schwenklager (14) vereinigt sind, jedes der ersten und zweiten Jochteile (12) bildend eine Innenfläche, wobei die Innenflächen um weniger als den Durchmesser genannter Linse voneinander beabstandet sind, jedes der Jochteile außerdem definierend eine gekrümmte Nut (16) innerhalb genannter Innenfläche zum Halten der Linse (20) ohne Druckbelastung, wobei die ersten und zweiten Verlängerungen (18) jeweils mit den ersten und zweiten Jochteilen (12) verbunden und ausreichend flexibel sind, so daß ein die Verlängerungen (18) zusammendrückender Fingerdruck die Jochteile (12) unter Freigabe der im Halter gehaltenen Linse aufspreizt.

2. Eine Kombination nach Anspruch 1, dadurch gekennzeichnet, daß genannte Verlängerungen (18) in einem Abstand im Bereich von 3,175 bis 4,445 cm (1,25 bis 1,75 Zoll) von dem Schwenklager (14) vereinigt sind.

3. Eine Kombination nach Anspruch 2, dadurch gekennzeichnet, daß die Vereinigungsstelle genannter Verlängerungen (18) von genanntem Schwenklager (14) um 3,4925 cm (1,375 Zoll) beabstandet ist.

4. Eine Kombination nach einem der Ansprüche 1 bis 3, dadurch gekennzeichnet, daß genannte Verlängerungen (18) an den Jochteilen (12) entgegengesetzten Enden durch integrale Endverbindungsmittel (19) verbunden sind und daß die Verlängerungen (18) bis zum gegenseitigen Kontakt im Bereich zwischen dem Schwenklager (14) und den integralen Endverbindungsmitteln (19) elastisch bewegbar sind, wobei die Kontaktzone einen Anschlag zur Begrenzung der maximalen Aufspreizung genannter erster und zweiter Jochteile (12) bildet.

5. Eine Kombination nach einem der Ansprüche 1 bis 4, dadurch gekennzeichnet, daß die ersten und zweiten Jochteile (12) eine durch die Achse genannter Verlängerungen gezogene Linie schneiden, um an genanntem Schwenklager (14) einen spitzen Winkel zu definieren.

6. Eine Kombination nach Anspruch 5, dadurch gekennzeichnet, daß der spitze Winkel im Bereich zwischen 10° und 50° liegt.

7. Eine Kombination nach einem der Ansprüche 1 bis 6, dadurch gekennzeichnet, daß der Linsenhalter (10) ein einstückig ausgebildetes Gerät ist.

8. Eine Kombination nach einem der Ansprüche 1 bis 7, dadurch gekennzeichnet, daß genannte Jochteile (12) derart zueinander gebogen sind, daß der Abstand zwischen den Enden der Jochteile kleiner als der Durchmesser der Linse (20) ist.

9. Eine Kombination aus einer Innenaugen-Linse (20) eines vorgegebenen Durchmessers und einem Linsenhalter (1) hierfür, genannter Linsenhalter aufweisend erste und zweite Jochteile (12), ein Schwenklager (14) und einen Handgriff (22); jedes der ersten und zweiten Jochteile (12) angebracht an genanntem Schwenklager (14) und in gegenseitigem Abstand, jedes der ersten und zweiten Jochteile (12) bildend eine Innenfläche, wobei die Innenflächen der ersten und zweiten Jochteile (12) um weniger als dem Durchmesser genannter Linse (20) voneinander beabstandet sind, jede der genannten Innenflächen außerdem definierend einen gekrümmten Kanal (16) zum Halten der Innenaugen-Linse (20) ohne Druckbelastung, genannter Handgriff (22) aufweisend erste und zweite Verlängerungen (18) der ersten und zweiten Jochteile, wobei genannte Verlängerungen (18) wenigstens abschnittsweise voneinander beabstandet sind und sich vom Schwenklager (14) aus über eine Strecke im Bereich von 3,175 bis 4,445 cm (1,25 bis 1,75 Zoll) erstrecken, wobei die Verlängerungen in einem Abstand vom Schwenklager (14) miteinander verbunden sind und einen Winkel mit den Jochteilen (12) bilden.

10. Eine Kombination nach einem der Ansprüche 1 bis 9, dadurch gekennzeichnet, daß genannte gekrümmte Schlitze oder Kanäle (16) einen Radius gleich dem Radius der Innenaugen-Linse (20) haben.

**Revendications**

1. Combinaison d'une lentille intraoculaire (20) de diamètre prédéterminée, et d'un porte-lentille (10) destiné à celle-ci, ce porte-lentille (10) comprenant un premier et un second élément de mâchoires (12), un point d'appui (14) et une poignée (22); chacun des premier et second éléments de mâchoires (12) étant relié au point d'appui (14) et espacé de l'autre élément, ces éléments de mâchoires (12) étant disposés de manière à tenir et à lâcher sélectivement la lentille, la poignée étant constituée par un premier et second prolongement (18) des premier et second éléments de mâchoires (12) au-delà du point d'appui (14), ces prolongements (18) étant parfaitement identiques l'un à l'autre et séparés l'un de l'autre par une distance prédéterminée à partir du point d'appui (14), ces prolongements (18) se reliant à une distance prédéterminée du point d'appui (14), chacun des premier et second éléments de mâchoires (12) définissant une face intérieure et ces faces intérieures étant séparées par une distance inférieure au diamètre de la lentille, chacun des éléments de mâchoires définissant une fente courbe (16) dans la face intérieure, pour retenir la lentille (20) sans appliquer de pression à celle-ci, les premier et second prolongements (18) étant couplés respectivement aux premier et second éléments de mâchoires (12), et suffisamment flexibles pour que la pression des doigts appliquée à ces prolongements (18) sépare les éléments de mâchoires (12) pour lâcher la lentille retenue dans le porte-lentille.

2. Combinaison selon la revendication 1, caractérisée en ce que les prolongements (18) se relient à une distance du point d'appui (14) se situant entre 3,175 et 4,445 cm.

3. Combinaison selon la revendication 2, caractérisée en ce que les prolongements (18) se rejoignent à une distance du point d'appui (14) égale à 3,4925 cm.

4. Combinaison selon l'une quelconque des revendications 1 à 3, caractérisée en ce qu'elle comprend des moyens de connecteur d'extrémité solidaires (19) reliant les prolongements (18) aux extrémités respectives opposées aux éléments de mâchoires (12), et en ce que les prolongements (18) sont mobiles de manière flexible pour venir mutuellement en contact entre le point d'appui (14) et les moyens de connecteur d'extrémité d'une seule pièce (19), la zone de contact formant une butée destinée à limiter la séparation maximum des premier et second éléments de mâchoires (12).

5. Combinaison selon l'une quelconque des revendications 1 à 4, caractérisée en ce que les premier et second éléments de mâchoires (12) se coupent suivant une ligne passant par l'axe des prolongements, de manière à former un angle aigu au point d'appui (14).

6. Combinaison selon la revendication 5, caractérisée en ce que l'angle aigu se situe entre dix degrés et cinquante degrés.

7. Combinaison selon l'une quelconque des revendications 1 à 6, caractérisée en ce que le porte-lentille (10) est un appareil d'une seule pièce.

8. Combinaison selon l'une quelconque des revendications 1 à 7, caractérisée en ce que les éléments de mâchoires (12) sont courbés l'un vers l'autre de façon que la distance entre les extrémités des éléments de mâchoires soit inférieure au diamètre de la lentille (20).

9. Combinaison d'une lentille intraoculaire (20) de diamètre prédéterminé, et d'un porte-lentille (10) destiné à celle-ci, le porte-lentille comprenant un premier et un second élément de mâchoires (12), un point d'appui (14), et une poignée (22); chacun des premier et second éléments de mâchoires (12) étant relié au point d'appui (14) et espacé de l'autre élément, chacun des premier et second éléments de mâchoires (12) définissant une face intérieure, les faces intérieures de ces premier et second éléments de mâchoires (12) étant séparées par une distance inférieure au diamètre de la lentille intraoculaire (20), chacune des faces intérieures définissant, en outre, un passage courbe (16) retenant la lentille intraoculaire (20) sans lui appliquer de pression, la poignée (22) étant constituée par un premier et second prolongement (18) des premier et second éléments de mâchoires, ces prolongements (18) comportant au moins certaines parties espacées l'une de l'autre et s'écartant du point d'appui (14) d'une distance se situant entre 3,175 et 4,445 cm, ces prolongements se rejoignant à une certaine distance du point d'appui (14) et formant un certain angle avec les éléments de mâchoires (12).

10. Combinaison selon l'une quelconque des revendications 1 à 9, caractérisée en ce que les fentes ou passages courbes (16) présentent un rayon égal au rayon de la lentille intraoculaire (20).

Fig. 1

Fig. 2

Fig. 3

Fig. 4

0 065 541